Europäisches Patentamt
European Patent Office
Office européen des brevets

(11) Publication number: **0 061 283 A1**

(12) **EUROPEAN PATENT APPLICATION**

(21) Application number: 82301322.2

(22) Date of filing: 16.03.82

(51) Int. Cl.³: C 07 H 19/08
C 07 H 19/06, A 61 K 31/70

(30) Priority: 20.03.81 GB 8108755
02.05.81 GB 8113618

(43) Date of publication of application:
29.09.82 Bulletin 82/39

(84) Designated Contracting States:
BE CH DE FR GB IT LI NL SE

(71) Applicant: BEECHAM GROUP PLC
Beecham House Great West Road
Brentford Middlesex(GB)

(72) Inventor: Harnden, Michael Raymond
47 Guildford Road
Horsham Sussex(GB)

(72) Inventor: Mock, Graham Andrew
34 Arundel Road
Sands High Wycombe Buckinghamshire(GB)

(74) Representative: Russell, Brian John et al,
European Patent Attorney Beecham Pharmaceuticals
Great Burgh Yew Tree Bottom Road
Epsom Surrey, KT18 5XQ(GB)

(54) **Antiviral agents, their preparation and use.**

(57) Compounds of formula (I):

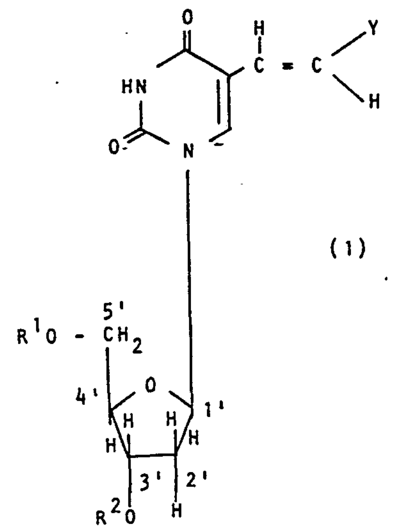

(I)

in which Y is a halogen atom, preferably a bromine atom, each of $R^1$ and $R^2$ is a hydrogen atom or an acyl radical of the formula

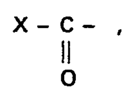

in which X is a $C_{1-6}$ alkyl group or a carboxy group of the formula

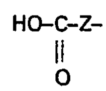

in which Z is a branched- or straight-chain alkylene radical having from 1 to 4 carbon atoms in the chain, with the proviso that $R^1$ and $R^2$ are not simultaneously hydrogen atoms or acetyl groups;
are useful in the treatment of viral infection, particularly those caused by herpes viruses.

EP 0 061 283 A1

# ANTIVIRAL AGENTS, THEIR PREPARATION AND USE

This invention relates to certain deoxyuridine compounds which have antiviral activity.

U.K. Patent Specification No. 1,601,020 discloses 5-(2-halogenovinyl)-2'-deoxyuridines which have antiviral activity selective against herpes virus. Recently, the 3',5'-diacetate of one of these uridines, E-5-(2-bromovinyl)-2'-deoxyuridine, has been disclosed.

[4th International Round Table, University of Antwerp, 4th - 6th February 1981, "Nucleosides" - Synthesis of substituted 5-vinyl pyrimidines (Barwolff)]

but no antiviral activity has been demonstrated for this compound.

We have now found a group of mono- and di-esters of 5-(2-halogenovinyl)-2'-deoxyuridine which have excellent antiviral activity, and which are useful in the treatment of infections caused by herpes viruses, such as herpes simplex type 1, herpes simplex type 2 and varicella. The esters also have excellent water solubility which makes them particularly useful for formulating pharmaceutical compositions.

According to the present invention there is provided an ester of the formula (I):

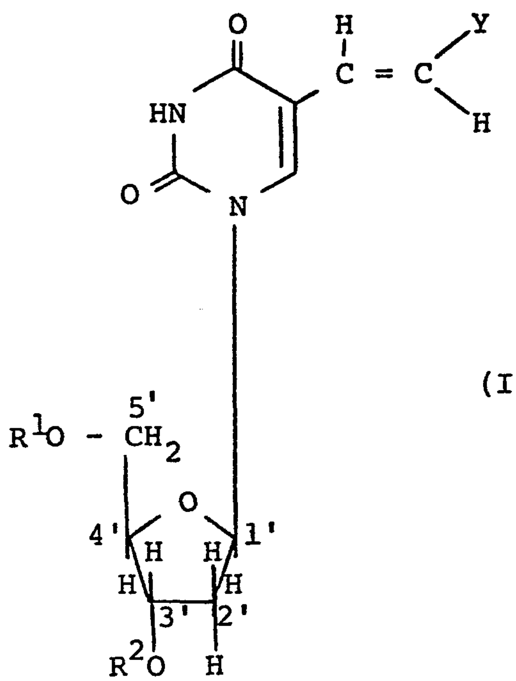

(I)

in which Y is a halogen atom, preferably a bromine atom, each of $R^1$ and $R^2$ is a hydrogen atom or an acyl radical of the formula $X - \underset{\substack{\| \\ O}}{C} -$ , in which X is a $C_{1-6}$ alkyl group or a carboxy group of the formula $HO-\underset{\substack{\| \\ O}}{C}-Z-$

in which Z is a branched- or straight-chain alkylene radical having from 1 to 4 carbon atoms in the chain, with the proviso that $R^1$ and $R^2$ are not simultaneously hydrogen atoms or acetyl groups.

Preferably, Z represents a $-(CH_2)_n-$ group in which n is from 1 to 4, suitably 2 or 3. When Z is a branched chain alkylene radical, the branching suitable comprises one or more lower alkyl groups, preferably methyl groups attached to carbon atoms in the chain. Examples of such branched radicals are:

$$-\underset{CH_3}{\overset{CH_3}{C}}-CH_2- ; \quad -CH_2-\underset{CH_3}{\overset{CH_3}{C}}- ; \quad -\underset{CH_3}{CH}-\underset{CH_3}{CH}- ;$$

$$-\underset{CH_3}{\overset{CH_3}{\underset{|}{\overset{|}{C}}}}-CH_2CH_2- \quad ; \quad -CH_2CH_2-\underset{CH_3}{\overset{CH_3}{\underset{|}{\overset{|}{C}}}} \quad ; \quad -CH_2-\underset{CH_3}{\overset{CH_3}{\underset{|}{\overset{|}{C}}}}-CH_2-$$

When $R^1$ and $R^2$ are simultaneously acyl radicals, the groups X in both $R^1$ and $R^2$ are preferably simultaneously $C_{1-6}$ alkyl or simultaneously $HO-\underset{\underset{O}{\|}}{C}-Z-$.

Suitably, when $R^1$ and $R^2$ are simultaneously acyl radicals they are identical.

Preferred $C_{1-6}$ alkyl groups for X are methyl, n-propyl and n-butyl.

3',5'-Diesters of formula (I) in which $R^1$ and $R^2$ are simultaneously $X - \underset{\underset{O}{\|}}{C} -$ where X is as defined in formula (I), may be prepared by treating a compound of formula (II):

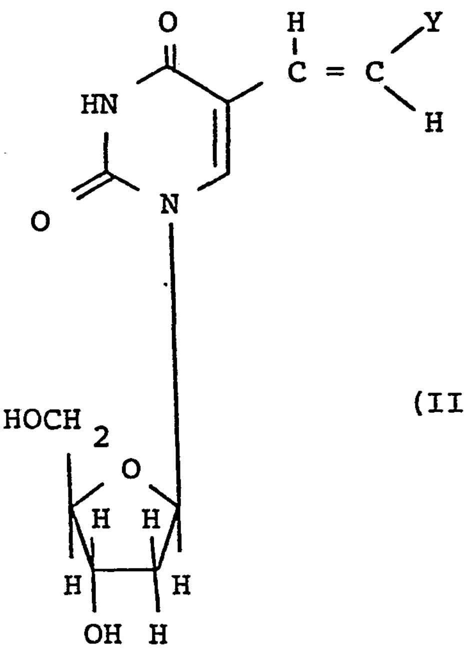

(II)

with an excess of an acylating agent containing the $X-\underset{\underset{O}{\|}}{C}-$ group. A preferred acylating agent is an acid anhydride of formula $(X-\underset{\underset{O}{\|}}{C}-)_2O$ or an acid chloride of formula $X-\underset{\underset{O}{\|}}{C}-Cl$.

The reaction is suitably carried out in an anhydrous polar organic solvent, preferably anhydrous pyridine, at room temperature.

The product is preferably purified chromatographically by, for example, column chromatography on silica gel.

5'-Monoesters of formula (I) in which $R^1$ is a $X-\underset{\underset{O}{\|}}{C}-$ group, where X is defined in formula (I), and $R^2$ is a hydrogen atom, may also be prepared in a similar manner as the diester preparation described above, but using approximately 1:1 molar ratio of acylating agent to starting compound of formula (II).

The reaction is suitably carried out in anhydrous pyridine at room temperature, and the product is preferably purified chromatographically on silica gel.

3'-Monoesters of formula (I) in which $R^2$ is an $X-\underset{\underset{O}{\|}}{C}-$ group, where X is as defined in formula (I), and $R^1$ is a hydrogen atom, may be prepared by heating a 5'-trityl compound of formula (III),

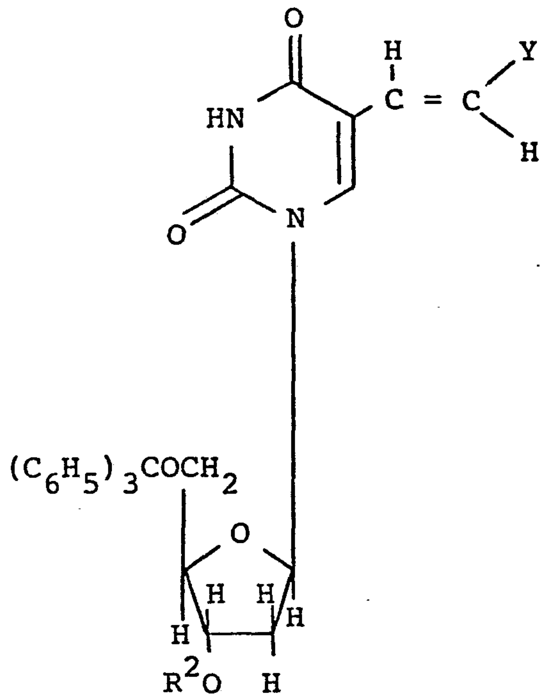

(III)

in which Y is as defined in formula (I), and $R^2$ is an acyl group as defined in formula (I), in the presence of an acid. If a strong acid is used, the heating is preferably carried out at a low temperature, and if a weak acid is used, the heating is preferably carried out at a high temperature.

A preferred acid is acetic acid.

The product may be purified chromatographically, suitably by thick layer chromatography.

The compound of formula (III) may itself be prepared by treating a 5'-trityl compound of formula (IV):

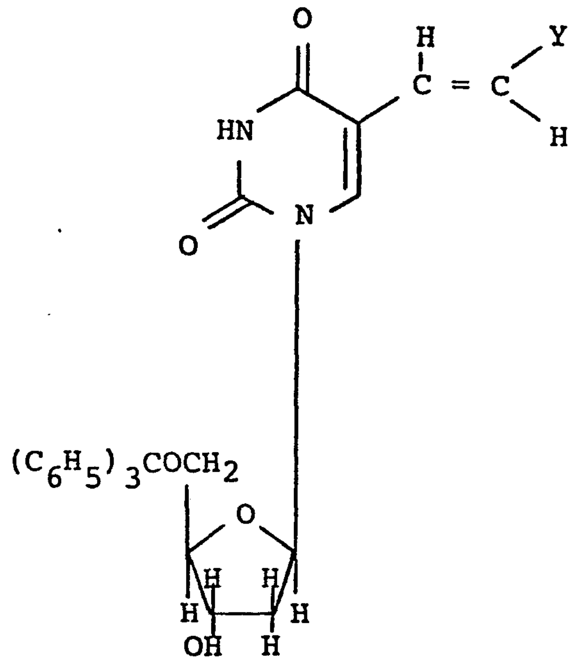

(IV)

with an acylating agent containing the $X-\underset{\overset{\|}{O}}{C}-$ group, preferably an acid anhydride of formula $(X-\underset{\overset{\|}{O}}{C}-)_2O$ or an acid chloride of formula $X-\underset{\overset{\|}{O}}{C}-Cl$.

The reaction conditions are similar to those described above for the preparation of the 3',5'-diesters and 5'-monoesters.

The compounds of formulae (III) and (IV) are important intermediates in the preparation of the 3'-monoesters and are themselves novel compounds which form a further aspect of the present invention.

The compounds of formula (IV) may be prepared by treating a compound of formula (II) as defined above, with a triphenyl halo methane compound, preferably triphenyl chloromethane. The reaction is suitably carried out in an anhydrous polar organic solvent, preferably at room temperature.

The product may be purified by column chromatography on silica gel.

The compounds of formula (I) may be formulated for use in a pharmaceutical composition. Accordingly, in a further aspect of the invention, there is provided a pharmaceutical composition which comprises a compound of the formula (I) together with a pharmaceutically acceptable carrier or excipient.

Compositions which may be given by the oral route may be compounded in the form of syrups, tablets and capsules. When the composition is in the form of a tablet, any pharmaceutical carrier suitable for formulating such solid compositions may be used, for example magnesium stearate, starch, lactose, glucose, rice, flour and chalk. The composition may also be in the form of an ingestible capsule, for example of gelatin, to contain the compound, or in the form of a syrup, a solution or a suspension. Suitable liquid pharmaceutical carriers include ethyl alcohol, glycerine, saline and water to which flavouring or colouring agents may be added to form syrups. The compounds may also be presented with a sterile liquid carrier for injection.

The composition may also be formulated for topical application to the skin or eyes.

For topical application to the skin, the compounds of the invention may be made up into a cream, lotion or ointment. These formulations may be conventional formulations well known in the art, for example, as described in standard books of pharmaceutics and cosmetics, such as Harry's Cosmeticology published by Leonard Hill Books, and the British Pharmacopaeia.

The composition for application to the eyes may be a conventional eye-drop composition well known in the art.

Preferably, the compositions of this invention are in unit dosage form or in some other form that the patient may administer to himself a single dose. A suitable dosage unit might contain from 50 mg to 1 g of active ingredient, for example 100 to 500 mg. Such doses may be administered 1 to 4 times a day or more usually 2 or 3 times a day. The effective dose of compound depends on the particular compound employed, but is in general in the range of from 1.0 mg/kg/day to 20 mg/kg of body weight per day or more usually 2.0 mg/kg/day to 10 mg/kg/day.

In a further aspect of the invention, there is provided a method of treating viral infections in human and non-human animals, which comprises administering to the sufferer an effective amount of a compound of formula (I).

The following Examples illustrate the invention.

Examples 1 and 2

General Method for the Preparation of E-5-(2-Bromovinyl)-2'-deoxyuridine-3',5'-diesters

To a solution of E-5-(2-bromovinyl)-2'-deoxyuridine (1 g, 3 mmol) in anhydrous pyridine (10 ml) at 5$^{o}$C was added the appropriate acid anhydride or acid chloride (15 mmol), dropwise over 4 minutes. The reaction mixture was stirred at room temperature for 20 hours and then poured into dichloromethane/water (80:50 ml). The aqueous layer was separated and further extracted with dichloromethane (1 x 80 ml, 1 x 50 ml). The separate dichloromethane extracts were washed with 1M hydrochloric acid (50 ml), cold saturated sodium hydrogen carbonate solution (50 ml), saturated sodium chloride solution (50 ml) and then combined and dried ($Na_2SO_4$).

The 3',5'-dibutyrate (Example 1) and divalerate esters (Example 2) were isolated as brown oils and were purified by column chromatgraphy on silica gel, elution with ethyl acetate/hexane (85:15).

The yields of the two compounds are as follows:

| | Acylating Agent | Yield % |
|---|---|---|
| Example 1 | | |
| E-5-(2-Bromovinyl)-3',5'-di-O-butyryl-2'-deoxyuridine | $CH_3(CH_2)_2COCl$ | 64 |
| Example 2 | | |
| E-5-(2-Bromovinyl)-3',5'-di-O-valeryl-2'-deoxyuridine | $CH_3(CH_2)_3COCl$ | 56 |

Further characterising data for Examples 1 and 2 is as follows.

Example 1

E-5-(2-Bromovinyl)-3',5'-di-O-butyryl-2'-deoxyuridine

T.l.c.:$R_f^a$ = 0.52 in ethyl acetate/hexane (1:1);
ν max (1<KBr)3430 (NH), 1743, 1712, 1678 (C=O) $cm^{-1}$;
λ max (MeOH) 249 nm (ε 13790);
$^1$Hn.m.r. ($CDCl_3$) δ 1.0 (6H,t,J 6.7Hz, 2 x $CH_3$);
1.7 (4H,q,J 6.7Hz, 2 x $\underline{CH_2}CH_3$),
2.33 (4H,t,J=6.7Hz, 2 x $CH_2CO$) superimposes on a multiplet (2H,2'-$CH_2$), 4.33 (3H,m,4'-CH,5'-$CH_2$), 5.2 (1H,m,3'-CH),
6.26 (1H,dd,$J_{AX}$=8Hz,$J_{BX}$=6.7Hz,1'-CH),
6.66 (1H,d,J=13.3Hz,<u>CH</u>=CHBr), 7.43 (1H,d,J=13.3Hz,CH=<u>CH</u>Br),
7.5 (1H,s,6-CH), 9.0 (1H,s,NH);

m/e 472, 474 ($M^+$, 0.8%), 257 (12), 168 (28), 81 (100).

Analysis

| | | |
|---|---|---|
| Found: | C, 48.46; H, 5.44; N, 6.29. | $C_{19}H_{25}N_2O_7Br$ |
| requires: | C, 48,21; H, 5.32; N, 5.92% | |

a Note: $R_f$ values were measured on Merck silica gel 60F precoated plates, layer thickness 0.5 mm.

Example 2

E-5-(2-Bromovinyl)-3',5'-di-O-valeryl-2'-deoxyuridine

T.l.c.: $R_f$= 0.55 in ethyl acetate/hexane (1:1);
ν max (KBr) 3440 (NH), 1740, 1712, 1680 (C=O) $cm^{-1}$;
λ max (MeOH) 291.5 nm (ε 11,700), 249 nm (ε 14,100);
$^1$Hn.m.r. ($CDCl_3$) δ 0.9 (6H,t,J=7Hz,2 x $CH_3$);
1.45 (8H,m,$C_2H_4CH_3$), 2.32 (4H,t,J=7Hz,2 x $CH_2CO$)
superimposed on a multiplet (2H,2'-$CH_2$),
4.3 (3H,m,4'-CH,5'-$CH_2$) 5.18 (1H,m,3'-CH),
6.26 (1H,dd,$J_{AX}$=8Hz,$J_{BX}$=6.7Hz,1'-CH),
6.62 (1H,d,J=13.5Hz,CH=CHBr), 7.4 (1H,d,J=13.5Hz, CH=CHBr),
7.5 (1H,s,6-CH), 9.32 (1H,s,NH);

m/e 500, 502 ($M^+$, 0.4%), 285 (6), 137 (16), 81 (100).

Analysis

| | | |
|---|---|---|
| Found: | C, 51.01; H, 5.83; N, 5.52. | $C_{21}H_{28}N_2O_7Br$ |
| requires: | C, 50.41; H, 5.64; N, 5.56% | |

Example 3

Preparation of E-5-(2-Bromovinyl)-5'-O-trityl-2-deoxyuridine (Compound of formula (IV) wherein Y is Br)

A solution of E-5-(2-bromovinyl)-2'-deoxyuridine (0.5 g, 1.5 mmol) and triphenylchloromethane(0.6 g, 1.53 mmol) in anhydrous pyridine (5 ml) was stirred at room temperature for 20 hours. The brown reaction mixture was poured into dichloromethane/water (50:50 ml) and the aqueous layer was separated and further extracted with dichloromethane (2 x 50 ml). The separate dichloromethane extracts were washed with cold, saturated sodium hydrogen carbonate solution (50 ml), saturated sodium chloride solution (50 ml), and then combined and dried ($Na_2SO_4$). Evaporation of the solvent in vacuo, followed by codistillation of the residue with carbon tetrachloride (50 ml), afforded a yellow oil (1.1 g) which was purified by column chromatography on silica gel (30 g). Elution with ethyl acetate/hexane (85:15) (100 ml) gave the 5'-O-trityluridine (0.69 g, 80%). A sample recrystallised from dichloromethane/hexane had m.p. 165- 67°C.

T.l.c.: $R_f$ = 0.5 in ethyl acetate;
ν max (KBr) 3430 (NH,OH), 1695 (C=O) $cm^{-1}$;
λ max (EtOH) 294 nm (ε 10,500); 250 nm (ε 13,100);
$^1$Hn.m.r. (DMSO $d_6$) δ 2.2 (2H,m,2'-$CH_2$), 3.2 (2H,m,5'-$CH_2$), 3.84 (1H,m,4'-CH), 4.25 (1H,m,3'-CH), 5.28 (1H,m,3'-CH), 6.12 (1H,t,J=7Hz,1'-CH), 6.44 (1H,d,J=14Hz,CH=CHBr), 7.4 (16H,m,$(C_6H_5)_3$C,CH=CHBr), 7.72 (1H,s,6-CH), 11.62 (1H,m,NH);

m/e 243 (22%), 183 (69), 165 (45), 154 (27), 105 (100).

Analysis

Found: C, 62.22; H, 4.86; N, 4.57. $C_{30}H_{27}N_2O_5Br$
requires: C, 62.65; H, 4.73; N, 4.87%

Examples 4 and 5

Preparation of E-5-(2-bromovinyl)-5'-O- trityl-2'-deoxyuridine-3'-O-esters (compounds of formula (III) wherein Y is Br)

To a solution of E-5-(2-bromovinyl)-5'-O-trityl-2'-deoxyuridine (0.6 g, 1 mmol) in anhydrous pyridine (10 ml) was added the appropriate acid anhydride or acid chloride (1.5 mmol) dropwise. After stirring at room temperature for 20 hours, an additional 0.85 mmol of acylating agent was added and stirring was continued for a further 4 hours. The reaction mixture was then poured into dichloromethane/ water (50:50 ml) and the aqueous layer was separated and further extracted with dichloromethane (2 x 30 ml). The separate dichloromethane extracts were washed with cold, saturated sodium hydrogen carbonate solution (50 ml), saturated sodium chloride solution (50 ml), and then combined and dried ($Na_2SO_4$). Evaporation of the solvent *in vacuo* gave an oil which was purified by column chromatography (silica gel (3.0 g), elution with ethyl acetate/ hexane (70:30) (140 ml) ).

The following two compounds were prepared by this method.

| | Acylating Agent | Yield % |
|---|---|---|
| Example 4 | | |
| 3'-O-Acetyl-E-5-(2-bromovinyl)-5'-O-trityl-2'-deoxyuridine | $(CH_3CO)_2O$ | 73 |
| Example 5 | | |
| E-5-(2-Bromovinyl-5'-O-trityl-3'-O-valeryl-2'-deoxyuridine | $CH_3(CH_2)_3COCl$ | 74 |

Further characterising data for Examples 4 and 5 are as follows.

Example 4

3'-O-Acetyl-E-5-(2-bromovinyl)-2'-deoxy-5'-O-trityl-2'-deoxyuridine

T.l.c. Rf = 0.69 in ethyl acetate.

ν max (KBr) 3430 (NH,OH), 1740, 1715, 1695 (C=O) $cm^{-1}$;

λ max (EtOH) 293 nm (ε 11,200), 250 nm (ε 14,000);

$^1$Hn.m.r. ($CDCl_3$) δ 2.1 (3H,s, $CH_3CO$), 2.5 (2H,m,2'-$CH_2$), 3.5 (2H,m,5'-$CH_2$), 4.15 (1H,m,4'-CH), 5.48 (1H,m,3'-CH), 5.79 (1H,d,J=13.5Hz, <u>CH</u>=CHBr), 6.38 (1H,dd,$J_{AX}$=8Hz, $J_{BX}$=6Hz, 1'-CH), 7.43 (16H,m,($C_6\underline{H}_5$)$_3$, CH = <u>CH</u>Br), 7.66 (1H,s, 6-CH);

m/e 616, 618 ($M^+$, 1.5%), 243 (100).

Analysis

Found: C, 62.45; H, 4.88; N, 4.51. $C_{32}H_{29}N_2O_6Br$ requires: C, 62.24; H, 4.73; N, 4.53%.

Example 5

E-5-(2-Bromovinyl)-5'-O-trityl-3'-O-valeryl-2'-deoxyuridine

T.l.c. Rf = 0.77 in ethyl acetate.
ν max (KBr) 3440 (NH, OH), 1730, 1720, 1665 (C=O) $cm^{-1}$;
λ max (EtOH) 293nm(ε 9430), 249.5 nm (ε 12,120);
$^1H$ n.m.r. ($CDCl_3$) δ 0.9 (3H, t, J=6.2Hz, $C\underline{H}_3CH_2$),
1.5 (4H, m, $\underline{C_2H_4}CH_3$), 2.4 (4H, m, $CH_2CO$, 2'-$CH_2$),
3.5 (2H, m, 5'-$CH_2$), 4.16 (1H, m, 4'-CH), 5.5 (1H, m. 3'-CH),
5.75 (1H, d, J=13.3Hz, $\underline{CH}$=CHBr), 6.38 (1H, t, J=6.7Hz,1'-CH),
7.35 (16H, m, $(C_6\underline{H}_5)_3C$, CH=$\underline{CH}$Br), 7.65 (1H, s, 6-CH);

m/e 658, 660 ($M^+$, 3%), 243 (100), 165 (100).

Analysis

| | | |
|---|---|---|
| Found: | C, 63.77; H, 5.40; N, 4.06. | $C_{35}H_{35}N_2O_6Br$ |
| requires: | C, 63.73; H, 5.35; N, 4.25%. | |

Examples 6 and 7

General Method for the Preparation of E-5-(2-Bromovinyl)-2'-deoxyuridine-3'-O-esters

A solution of the E-5-(2-bromovinyl)-5'-O-trityl-2'-deoxyuridine-3'-O-ester (0.25 mmol) in acetic acid (2 ml) was heated under reflux for 6 minutes. The solvent was removed *in vacuo* and the residue purified by preparative thick layer chromatography, elution with ethyl acetate/hexane (75:25).

The following two compounds were prepared by this method.

| | Yield % |
|---|---|
| Example 6 | |
| 3'-O-Acetyl-E-5-(2-bromovinyl)-2'-deoxyuridine | 78 |
| Example 7 | |
| E-5-(2-Bromovinyl)-3'-O-valeryl-2'-deoxyuridine | 74 |

Further characterising data for Examples 6 and 7 are as follows.

Example 6

3'-O-Acetyl-E-5-(2-bromovinyl)-2'-deoxyuridine

T.l.c. Rf = 0.6 in ethyl acetate.
ν max (KBr) 3415, 3250 (NH, OH), 1745, 1695, 1680 (C=O) $cm^{-1}$;
λ max (EtOH) 292 nm (ε 12,000), 249.5 nm (ε 14,200);
$^1H$ n.m.r. (DMSO $d_6$) δ 2.05 (3H, s, $CH_3CO$),
2.35 (2H, m, 2'-$CH_2$), 3.67 (2H, m, 5'-$CH_2$),
4.03 (1H, m, 4'-CH), 5.24 (2H, m, 3'-CH, 5'-OH),
6.15 (1H, t, J=6.4Hz, 1'-CH), 6.84 (1H, d, J=13.8Hz, <u>CH</u>=CHBr),
7.28 (1H, d, J=13.8Hz, CH=<u>CH</u>Br), 8.08 (1H, s, 6-CH),
11.6 (1H, s, NH);

m/e 374, 376 ($M^+$, 1%), 243 (7.5), 216 (8), 137 (77), 99 (99), 69 (100).

Analysis

Found: C, 41.54; H, 3.99; N, 7.13. $C_{13}H_{15}N_2O_6Br$
requires: C, 41.62; H, 4.03; N, 7.47%.

Example 7

E-5-(2-Bromovinyl)-3'-O-valeryl-2'-deoxyuridine

T.l.c. Rf = 0.57 in ethyl acetate.
ν max (KBr) 3460, 3260 (NH, OH), 1720, 1710, 1682 (C=O) $cm^{-1}$;
λ max (EtOH) 292.5 nm (ε 11,880), 249.5 nm (ε 14.310);
$^1H$ n.m.r. (DMSO $d_6$) δ 0.9 (3H, t, J=6.4Hz, $C\underline{H}_3CH_2$),
1.45 (4H, m, $C_2\underline{H}_4CH_3$), 2.33 (4H, m, $CH_2CO$, 2'-$CH_2$),
3.65 (2H, m, 5'-$CH_2$), 4.03 (1H, m, 4'-CH),
5.22 (2H, m, 3'-CH, 5'-OH), 6.16 (1H, t, J=6.4Hz, 1'-CH),
6.84 (1H, d, J=13.8Hz, $\underline{CH}$=CHBr), 7.3 (1H, d, J=13.8Hz,CH=$\underline{CH}$Br),
8.1 (1H, s, 6-CH), 11.58 (1H, s, NH);

m/e 416/418 ($M^+$, 0.5%), 201 (10), 137 (68), 99 (100).

Analysis

| | | |
|---|---|---|
| Found: | C, 46.29; H, 4.97; N, 6.49. | $C_{16}H_{21}N_2O_6Br$ |
| requires: | C, 46.06; H, 5.07; N, 6.71%. | |

Examples 8 and 9

General Method for the Preparation of E-5-(2-Bromovinyl)-2'-deoxyuridine-5'-O-esters

To a solution of E-5-(2-bromovinyl)-2'-deoxyuridine (0.2 g, 0.6 mmol) in anhydrous pyridine (3 ml) was added the appropriate acid anhydride or acid chloride (0.6 mmol), dropwise. The reaction mixture was stirred at room temperature for 1.5 hours and then poured into dichloromethane/water (20:20 ml). The aqueous layer was separated and further extracted with dichloromethane (2 x 20 ml). The separate dichloromethane extracts were washed with cold, saturated sodium hydrogen carbonate solution (20 ml), saturated sodium chloride solution (20 ml), and then combined and dried ($Na_2SO_4$). Evaporation of the solvent *in vacuo* gave an oil which was purified by column chromatography (silica gel (10 g), elution with ethyl acetate/hexane (85:15) (150 ml) ).

The following two compounds were prepared by this method.

| | Acylating Agent | Yield % |
|---|---|---|
| Example 8 | | |
| 5'-O-Acetyl-E-5-(2-bromovinyl)-2'-deoxyuridine | $(CH_3CO)_2O$ | 24 |
| Example 9 | | |
| E-5-(2-Bromovinyl)-5'-O-valeryl-2'-deoxyuridine | $CH_3(CH_2)_3COCl$ | 60 |

Further characterising data for Examples 8 and 9 are as follows.

Example 8

5'-O-Acetyl-E-5-(2-bromovinyl)-2'-deoxyuridine

T.l.c. Rf = 0.38 in ethyl acetate.
ν max (KBr) 3420, 3220 (NH, OH), 1738, 1712, 1670 (C=O) $cm^{-1}$;
λ max (EtOH)
$^1H$ n.m.r. (DMSO $d_6$) δ 2.0 (3H, s, $CH_3CO$), 2.18 (2H, m, 2'-$CH_2$),
3.88 (1H, m, 4'-CH), 4.2 (3H, m, 3'-CH, 5'-$CH_2$),
5.4 (1H, d, J=4.5Hz, 3'-OH), 6.13 (1H, t, J=6.9Hz, 1'-CH),
6.91 (1H, d, J=14Hz, CH=CHBr), 7.29 (1H, d, J=14Hz, CH=CHBr),
7.78 (1H, s, 6-CH), 11.62 (1H, s, NH).

Example 9

E-5-(2-Bromovinyl)-5'-O-valeryl-2'-deoxyuridine

T.l.c. Rf = 0.52 in ethyl acetate.
ν max (KBr) 3420, 3215 (NH, OH), 1730, 1715, 1668 (C=O) $cm^{-1}$;
λ max (EtOH) 293.5 nm (ε 12,850), 250 nm (ε 15,140);
$^1H$ n.m.r. (DMSO $d_6$) δ 0.83 (3H, t, J=6.4Hz, $CH_3CH_2$),
1.38 (4H, m, $C_2H_4CH_3$), 2.35 (4H, m, $CH_2CO$, 2'-$CH_2$),
3.98 (1H, m, 4'-CH), 4.23 (3H, m, 3'-CH, 5'-$CH_2$),
5.4 (1H, s, 3'-OH), 6.17 (1H, t, J=6.4Hz, 1'-CH),
6.88 (1H, d, J=13.8Hz, CH=CHBr), 7.32 (1H, d, J=13.8Hz, CH=CHBr),
7.76 (1H, s, 6-CH), 11.59 (1H, s, NH).

Analysis

| | | |
|---|---|---|
| Found: | C,45.95; H, 4.74; N, 6.69. | $C_{16}H_{21}N_2O_6Br$ |
| requires: | C,46.06; H, 5.07; N, 6.71%. | |

Example 10

Preparation of E-5-(2-Bromovinyl)-3',5'-di-O-(3-carboxypropionyl)-2'-deoxyuridine monohydrate

A mixture of E-5-(2-bromovinyl)-2'-deoxyuridine (1g, 3 mmol), succinic anhydride (0.92 g, 9.2 mmol) and 4-dimethylaminopyridine (0.04g, 0.33 mmol) was stirred in anhydrous pyridine at room temperature for 20 hours. The solvent was removed *in vacuo* to give a brown oil (3 g) which was column chromatographed twice (silica gel, elution with dichloromethane/methanol (9:1) and then recrystallised from water to give the ω-diacid (0.35 g, 25%) as fine needles, m.p. 145-147°C;

t.l.c. Rf = 0.26 in dichloromethane/methanol (9:1).
ν max (KBr) 3415, 3190 (NH, OH), 1735, 1712, 1690 (C=O) $cm^{-1}$.
λ max (EtOH) 293 nm (ε 12,400), 249.5 (ε 14,790).
$^1H$ n.m.r. (DMSO $d_6$) δ 2.54 (10H, s, $(HO_2CC_2H_4CO)_2$, 2'-$CH_2$), 4.22 (3H, m, 4'-CH, 5'-$CH_2$), 5.2 (1H, m, 3'-CH), 6.12 (1H, t, J=7Hz, 1'-CH), 6.88 (1H, d, J=14Hz, CH=CHBr), 7.32 (1H, t, J=14Hz, CH=CHBr), 7.82 (1H, s, 6-CH), 11.66 (1H, m, NH, OH).

Analysis

| | | |
|---|---|---|
| Found: | C, 42.34; H, 4.29; N, 5.33. | $C_{19}H_{21}N_2O_{10}Br$ |
| requires: | C, 42.63; H, 4.33; N, 5.23%. | |

Example 11

(a) Preparation of E-5-(2-Bromovinyl)-3'-O-(3-Carboxypropionyl)-5'-O-trityl-2'-deoxyuridine

A mixture of E-5-(2-bromvinyl)-5'-O-trityl-2'-deoxyuridine (1 g, 1.7 mmol), succinic anhydride (0.5 g, 5 mmol) and 4-dimethylaminopyridine (0.015 g, 0.12 mmol) was stirred in anhydrous pyridine (15 ml), at room temperature, for 20h. A further 0.5 g (5 mmol) of succinic anhydride and 0.015 g (0.12 mmol) 4-dimethylaminopyridine was added and the reaction mixture was stirred at room temperature for 20h and then at 60°C for 4h. The solvent was removed under reduced pressure to give a brown oil (2.5 g) which was column chromatographed (silica gel, elution with dichloromethane/methanol (9:1)) to yield the ω-acid (0.8 g, 68%) as a foam.

t.l.c. Rf=0.64 in dichloromethane/methanol (17:3)

$^1$H.n.m.r. (DMSO $d_6$) δ 2.5 (6H,m, $HO_2CC_2H_4CO$, 2'-$CH_2$), 3.33 (2H,m,5'-$CH_2$), 4.1 (1H,m,4'-CH), 5.26 (1H,m,3'-CH), 6.2 (1H,t,J=6.7Hz,1'-CH), 6.38 (1H,d,J=13.3Hz,CH=CHBr), 7.2 (1H,d,J=13.3Hz,CH=CHBr), 7.33 (15H,s,$(C_6H_5)_3C$), 7.78 (1H,s,6-CH).

(b) Preparation of E-5-(2-bromovinyl)-3'-O-(3-carboxypropionyl)-2'-deoxyuridine

E-5-(2-bromovinyl)-3'-O-(3-carboxypropionyl)-5'-O-trityl-2'-deoxyuridine (0.6 g, 0.9 mmol) was heated under reflux in a mixture of acetic acid/water (8:2, 20 ml) for 15 minutes. The solvent was removed under reduced pressure to give a white solid which was column chromatographed (silica gel, elution with dichloromethane/methanol (9:1)) to yield the ω-monoacid as a foam (0.2 g). Recrystallisation

from water afforded the product as needles (0.145 g, 38%), m.p. 126-128°C.

t.l.c. Rf=0.36 in dichloromethane/methanol (17:3).

ν max (KBr) 3500, 3250(NH,OH), 1740, 1715, 1700 (C=O) $cm^{-1}$.
λ max (EtOH) 292.5 nm (ε 12,000), 249.5 nm (ε 14,100).
H.n.m.r. (DMSO $d_6$) δ 2.33 (2H,m,2'-$CH_2$), 2.53 (4H,s, $HO_2CC_2\underline{H}_4CO$), 3.33 (2H,m,$CO_2\underline{H}$,O$\underline{H}$), 3.66 (2H,m,5'-$CH_2$), 4.03 (1H,m,4'-CH), 5.25 (1H,m,3'-CH), 6.16 (1H,t,J=6.7Hz, 1'-CH), 6.83 (1H,d,J=13.3Hz, C$\underline{H}$=CHBr), 7.26 (1H,d,J=13.3Hz, CH=C$\underline{H}$Br), 8.08 (1H,s,6-CH), 11.58 (1H,m,NH).

m/e 216,218 (6%), 137 (100).

<u>Analysis</u>

Found: C, 39.75; H, 3.72; N, 6.61. $C_{15}H_{17}N_2O_8Br.H_2O$
requires: C, 39.92; H, 4.24; N, 6.20.

Example 12

Preparation of E-5-(2-bromovinyl)-5'-O-(3-carboxypropionyl)-2'-deoxyuridine

A mixture of E-5-(2-bromovinyl)-2'-deoxyuridine (0.5 g,, 1.5 mmol) and succinic anhydride (0.15 g, 1.5 mmol) was stirred in anhydrous pyridine (10 ml) at room temperature for 48 hours, and then at 100°C for 18 hours. The solvent was removed under reduced pressure to give a brown oil (0.7 g) which was column chromatographed (silica gel, elution with dichloromethane/methanol (9:1)) to yield the ω-monoacid (0.1 g) as a colourless foam. Recrystallisation from water afforded the product as needles (0.08 g , 12°C), m.p. 186-188°C.

t.l.c. Rf=0.31 in dichloromethane/methanol (17:3).

ν max (KBr) 3470, 3180 (NH, OH), 1735, 1725, 1685 (C=O) $cm^{-1}$; λ max (EtoH) 292.5 nm (ε 10,200), 249.5 nm (ε 12,000). H' n.m.r. (DMSO $d_6$) δ 2.2 (2H, m, 2'-$CH_2$), 2.48 (4H, s, $HO_2CC_2\underline{H}_4CO$), 3.1-4.3 (6H, m, 5'-$CH_2$, 3',4'-CH, $CO_2\underline{H}$, OH), 6.15 (1H, t, J=6.7Hz, 1'-CH), 6.9 (1H, d, J=13.3Hz, C<u>H</u>=CHBr), 7.3 (1H, d, J=13.3Hz, CH=C<u>H</u>Br), 7.76 (1H, s, 6-CH), 12.55 (1H, m, NH).

m/e 243 (9%), 219, 217 (16), 137 (100).

Analysis

Found: C, 41.84; H, 4.09; N, 6.12. $C_{15}H_{17}N_2O_8Br$ requires C, 41.59; H, 3.96; N, 6.47.

Example 13

Preparation of E-5-(2-Bromovinyl)-5'-O-pivaloyl-2'-deoxyuridine

To a solution of E-5-(2-bromovinyl)-2'-deoxyuridine (4.00 g, 12 mmol) in anhydrous pyridine (100 ml) at $0^{o}C$, pivaloyl chloride (12 mmol) was added dropwise and the reaction mixture stirred at $0^{o}C$ for 4 hours and then at $25^{o}C$ overnight. Iced water (25 ml) was added and the aqueous pyridine mixture evaporated under reduced pressure, yielding an oil. The oil was dissolved in ethyl acetate (300 ml) washed with saturated sodium bicarbonate solution (200 ml) and water (100 ml), dried (magnesium sulphate) and evaporated to a yellowish solid. The solid was triturated with diethyl ether/light petroleum (2:1, 75 ml) to remove the 3',5'-di-O-pivaloate and residual pivalic acid. The remaining solid was collected by filtration and dried to give the title compound (3.27 g, 65%) with mp 204-208$^{o}C$;

$\nu$ max (KBr) 1725, 1687, 1466, 1285, 1160, 1085 $cm^{-1}$;

$^{1}H$ nmr [$(CD_3)_2SO$] $\delta$ 1.15 (9H, s, 3 x $CH_3$), 1.90-2.30 (2H, m, 2'-$CH_2$), 3.50-4.50 (5H, m, 3'-CH, 4'-CH, 5'-CH, $D_2O$ exchangeable, OH), 6.10 (1H, m, 1'-CH), 6.80 (1H, d, CH=CHBr, J=14Hz), 7.25 (1H, d, CH=CHBr, J=14Hz), 7.65 (1H, s, $C^6H$), 11.65 (1H, br.s, $D_2O$ exchangeable, NH);

m/e (70eV) 416/418 ($M^+$, 2%).

Analysis Found: C, 46.17; H, 5.05; N, 6.65; Br, 19.22 %
$C_{16}H_{21}N_2O_6Br$ requires C, 46.04; H, 5.03; N, 6.71; Br, 19.18 %.

Example 14

Preparation of E-5-(2-bromovinyl)-3',5'-di-O-pivaloyl-2'-deoxyuridine

To a solution of E-5-(2-bromovinyl)-2'-deoxyuridine (1.00 g, 3 mmol) in anhydrous pyridine (50 ml) at $0^oC$, pivaloyl chloride (2 ml) was added and the resultant mixture stirred at $0^oC$ for 2 hours and then overnight at $25^oC$. Iced water (25 ml) was added and the aqueous pyridine mixture evaporated under reduced pressure, affording an oil. The oil was dissolved in chloroform (150 ml) and washed with saturated sodium bicarbonate solution (100 ml) and water (100 ml), dried (magnesium sulphate) and evaporated under reduced pressure to a yellow oil. Shortpath chromatography (10-20% acetone in n-hexane as eluent) followed by crystallisation from acetone-n-hexane mixtures gave the title compound (1.23 g, 83%), mp 135-137$^oC$;

$\nu$ max (KBr) 2970, 1730, 1707, 1675, 1475, 1278, 1145 $cm^{-1}$;

$^1H$ nmr (CDCl$_3$) $\delta$ 1.20 (18H, s, 6 x $CH_3$), 1.90-3.00 (2H, m, 2'-$CH_2$), 4.25-4.85 (3H, m, 4'-CH, 5'-$CH_2$), 5.20 (1H, br.d, 3'-CH), 6.35 (1H, dd, 1'-CH), 6.85 (1H, d, CH=CHBr, J=13Hz), 7.55 (2H, d, CH=CHBr, $C^6H$), 10.10 (1H, br.s, $D_2O$ exchangeable, NH);

m/e (70eV) 500/502 ($M^+$, 0.5%).

Analysis Found: C, 50.27; H, 5.74; N, 5.40; Br, 16.27 %
$C_{21}H_{29}N_2O_7Br$ requires: C, 50.29; H, 5.78; N, 5.58; Br, 15.97 %.

ANTIVIRAL ACTIVITY

In Vitro

Method

Vero (African Green Monkey Kidney) cells were grown to confluence in 6 well multidishes, each well being 3.5 cm in diameter. The cells were incubated with Herpes simplex type 1 virus (HFEM strain) and overlaid with 0.5 ml of 0.9% agarose (w/v) in maintenance medium containing the test compound at a range of concentrations from 50 μg/ml in half-log dilution steps. The virus infected cultures were then incubated at 37°C for 6 days before fixing in 4% formaldehyde solution and staining with carbolfuchsin. The dishes were then examined to find what concentration of test compound causing a 50% reduction in the number of virus plaques formed ($PDD_{50}$ value) and the minimum concentration of test compound which killed the cell monolayer, leaving a clear zone devoid of cells and virus plaques (MTD).

Results

| Example No. | $PDD_{50}$ | | MTD (µg/ml) |
|---|---|---|---|
| | µg/ml | µM | |
| 1 | 0.20 | 0.42 | > 100 |
| 2 | 0.20 | 0.40 | > 100 |
| 6 | 0.41 | 1.10 | > 100 |
| 7 | 0.18 | 0.43 | > 100 |
| 8 | 0.32 | 0.85 | > 100 |
| 9 | 0.16 | 0.38 | > 100 |
| 10 | 55.0 | 103.0 | > 100 |
| 11b | 0.18 | 0.40 | > 100 |
| 12 | 0.35 | 0.80 | > 100 |
| 13 | 0.52 | 1.3 | > 100 |
| 14 | 8.5 | 17.0 | > 100 |

CLAIMS

1. A compound of formula (I):

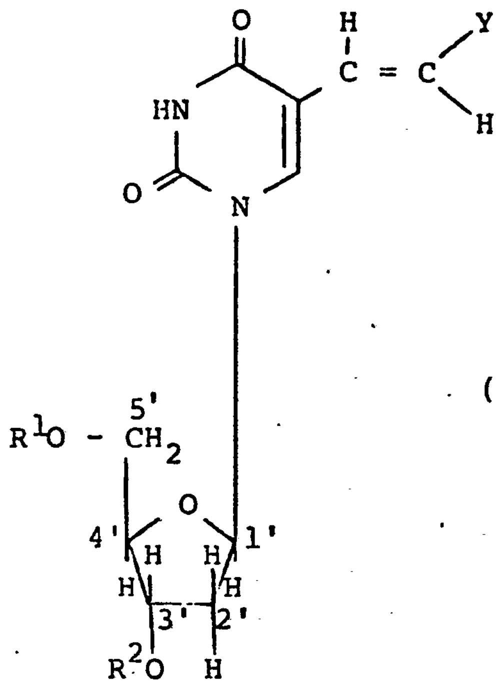

(I)

in which Y is a halogen atom, each of $R^1$ and $R^2$ is a hydrogen atom or an acyl radical of the formula $X-\underset{\underset{O}{\|}}{C}-$ , in which X is a $C_{1-6}$ alkyl group or a carboxy group of the formula $HO-\underset{\underset{O}{\|}}{C}-Z-$ , in which Z is a branched or straight-chain alkylene radical having from 1 to 4 carbon atoms in the chain, with the proviso that $R^1$ and $R^2$ are not simultaneously hydrogen atoms or acetyl groups.

2. A compound according to claim 1, in which Y represents a bromine atom.

3. A compound according to claim 1 or 2, in which Y represents a $-(CH_2)_n-$ group in which n is from 1 to 4.

4. A compound according to claim 1 or 2, in which $R^1$ and $R^2$ are identical.

5. A compound according to claim 1, selected from E-5-(2-bromovinyl)-3',5'-di-O-butyryl-2'-deoxyuridine, E-5-(2-bromovinyl)-3',5'-di-O-valeryl-2'-deoxyuridine, 3'-O-acetyl-E-5-(2-bromovinyl)-2'-deoxyuridine, E-5-(2-bromovinyl)-3'-O-valeryl-2'-deoxyuridine, 5'-O-acetyl-E-5-(2-bromovinyl)-2'-deoxyuridine, E-5-(2-bromovinyl)-5'-O-valeryl-2'-deoxyuridine, E-5-(2-bromovinyl)-3',5'-di-O-(3-carboxypropionyl)-2'-deoxyuridine monohydrate, E-5-(2-bromovinyl)-3'-O-(3-carboxypropionyl)-2'-deoxyuridine, E-5-(2-bromovinyl)-5'-O-(3-carboxypropionyl)-2'-deoxyuridine, E-5-(2-bromovinyl)-5'-O-pivaloyl-2'-deoxyuridine, and E-5-(2-bromovinyl)-3',5'-di-O-pivaloyl-2'-deoxyuridine.

6. A process for preparing a compound according to claim 1, which comprises treating a compound of formula (II):

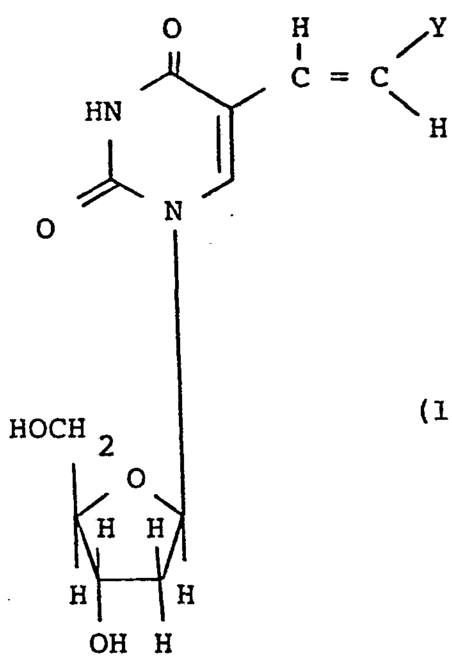

(II)

with an acylating agent containing the $X-\underset{\underset{O}{\|}}{C}-$ group.

7. A process according to claim 6, in which the acylating agent comprises an acid anhydride of formula $(X-\underset{\underset{O}{\|}}{C}-)_2O$ or an acid chloride of formula $X-\underset{\underset{O}{\|}}{C}-Cl$.

8. A process for preparing a 3'-monoester of formula (I) which comprises heating a 5'-trityl compound of formula (III):

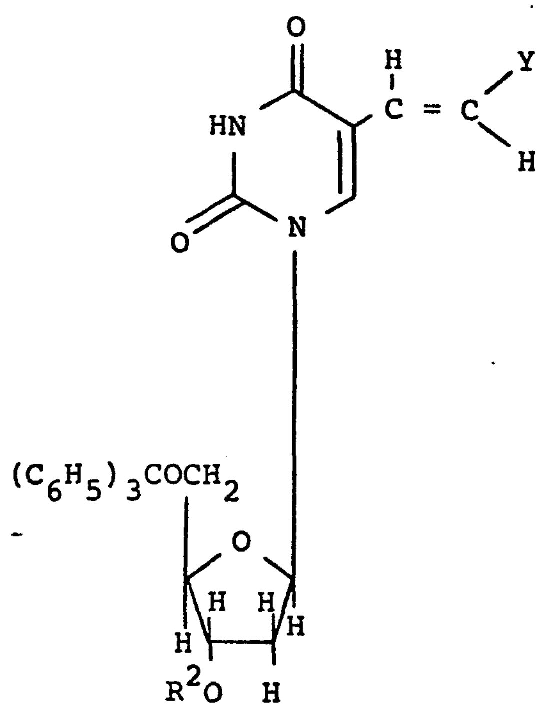

(III)

in which Y is as defined in formula (I), and $R^2$ is an acyl group as defined in formula (I).

9. A pharmaceutical composition comprising a compound according to any one of claims 1 to 5 in combination with a pharmaceutically acceptable carrier.

10. A compound according to any one of claims 1 to 5, or a composition according to claim 9, for use in the treatment of viral infections.

European Patent Office

# EUROPEAN SEARCH REPORT

0061283

Application number

EP 82 30 1322

| Category | DOCUMENTS CONSIDERED TO BE RELEVANT<br>Citation of document with indication, where appropriate, of relevant passages | Relevant to claim |
|---|---|---|
| Y | DE - A - 2 915 254 (UNIVERSITY OF BIRMINGHAM)<br>* pages 1,2 * | 1-10 |
| D | & GB - A - 2 060 604<br>-- | |
| Y | US - A - 3 323 994 (HOECHST)<br>* column 1, lines 14-36, 61-72 *<br>-- | 1-10 |
| Y | CHEMICAL ABSTRACTS, vol. 92, no. 23, June 9, 1980, abstract no. 198689x<br>COLUMBUS, Ohio (US)<br>& Nucl. Acid Chem. 1978, 1, 291-4<br>M.D. EDGE et al.:"3'-O-(carboxymethyl)thymidine. Carboxymethylation of thymidine at O-3'"<br>* the whole abstract *<br>----- | 8 |

CLASSIFICATION OF THE APPLICATION (Int. Cl. 3)

C 07 H 19/08
19/06
A 61 K 31/79

TECHNICAL FIELDS SEARCHED (Int.Cl. 3)

C 07 H 19/00
A 61 K 31/00

CATEGORY OF CITED DOCUMENTS

X: particularly relevant if taken alone
Y: particularly relevant if combined with another document of the same category
A: technological background
O: non-written disclosure
P: intermediate document
T: theory or principle underlying the invention
E: earlier patent document, but published on, or after the filing date
D: document cited in the application
L: document cited for other reasons

&: member of the same patent family, corresponding document

X The present search report has been drawn up for all claims

| Place of search | Date of completion of the search | Examiner |
|---|---|---|
| The Hague | 21-04-1982 | VERHULST |

EPO Form 1503.1 06.78